# EUROPEAN PATENT SPECIFICATION

(11) **EP 2 403 448 B1**
(45) Date of publication and mention of the grant of the patent: **24.12.2014**
(21) Application number: 09841223.2
(22) Date of filing: 06.03.2009
(51) Int. Cl.: A61F 13/505, A61F 13/56, A61F 13/474, A61F 13/493, A61F 13/47

(54) **STIFFENING ELEMENT AND ABSORBENT ARTICLE COMPRISING SUCH AN ELEMENT**
VERSTEIFUNGSELEMENT UND EIN SOLCHES ELEMENT UMFASSENDER SAUGFÄHIGER ARTIKEL
ÉLÉMENT RAIDISSEUR ET ARTICLE ABSORBANT COMPRENANT UN TEL ÉLÉMENT

(43) Date of publication of application: 11.01.2012
(73) Proprietor: SCA Hygiene Products AB, 405 03 Göteborg (SE)
(72) Inventor: DREVIK, Solgun, SE-435 35 Mölnlycke (SE); SAMUELSSON, Ann, SE-437 32 Lindome (SE)
(74) Representative: Zacco Sweden AB
(86) International application number: PCT/SE2009/050236
(87) International publication number: WO 2010/101499

(56) References cited:
- GB-A- 2 151 548
- US-A1- 2003 125 699
- US-A1- 2003 125 699
- US-A1- 2005 131 372
- US-B1- 6 423 043
- US-B1- 6 764 477

## Description

### TECHNICAL FIELD

This invention relates to a stiffening element for an absorbent article, such as a sanitary napkin, a panty liner or an incontinence protector, wherein the stiffening element is designed such as to, at least during use of the article, provide the article with a predetermined shape that enhances the fit of the article to a wearer's body The invention also relates to an absorbent article comprising such a stiffening element.

### BACKGROUND ART

Absorbent articles, such as sanitary napkins, incontinence guards, pantyliners, diapers etc., are well known in the art. An important function of absorbent articles is to prevent leakage of body exudates during use of the article. Generally, the article should fit well to the user and stay in place during use. This also enhances the user comfort.

With regard to at least sanitary napkins, incontinence guards and pantyliners, it is previously known to provide the article with stiff or elastic shaping elements that provide the article with a shape that improves the fitting and the ability to stay in place during use. In general, a stiff shaping element has the advantage that the shape of the article is predetermined and maintained during use. On the other hand, stiff shaping elements must be designed with particular care in order not to cause discomfort during use of the article. It is also well known to provide the underside of sanitary napkins and similar absorbent articles with fastening means, such as adhesives, for attachment to the user's garments.

WO 0117474 discloses an example of an absorbent article in the form of a sanitary napkin, a panty liner or an incontinence protector, wherein the rear portion of the article includes a longitudinally extending ridge-shaped elevation forming a stiff shaping element that partially extends between the wearer's buttocks during use of the article. This provides good protection against rearward leakage.

WO 98/22061 discloses an absorbent article in the form of a sanitary napkin having stiff front and crotch portions wherein the front portion is curved and inclined upwards, towards the user, with respect to the crotch portion. Further, the article has a narrow waist in the crotch portion allowing a high stiffness without causing discomfort. The desired stiffness is achieved by e.g. including a rigid shape-retaining, spoon-shaped, plastic or metal layer inside the article. The article according to WO 98/22061 is intended to be kept securely and comfortably in position against the body of the user during use, without the need for particular attachment means.

EP 1395218 discloses an adsorbent article in the form of a sanitary towel or incontinence pad comprising a combined, flat stiffening and absorbent element arranged inside the article, which element gives the article in different regions a predetermined two- or three-dimensional shape (including curvature, bowl-shape and a raised part between the buttocks of the wearer) during use of the article, i.e. when the article is affected by compressive forces generated by the thighs of the wearer. In similarity to WO 98/22061, the front and crotch portions are designed to allow anchoring of the article to thigh muscle tendons, which gives the article a good fit and stability in the fitted position.

Although the known absorbent articles with stiffening elements in many cases provide for a good fit, there still remains a need to further develop this type of absorbent articles.

### DISCLOSURE OF INVENTION

An object of this invention is to provide a stiff shaping element for an absorbent article, such as a sanitary napkin, that exhibits improved properties compared to conventional stiff shaping elements. This object is achieved by the element defined by the technical features contained in independent claim 1. The dependent claims contain advantageous embodiments, further developments and variants of the invention.

The invention concerns a stiffening element for an absorbent article, such as a sanitary napkin, a panty liner or an incontinence protector, wherein the stiffening element is designed such as to, at least during use of the article, provide the article with a predetermined shape that enhances the fit of the article to a wearer's body, said stiffening element having: a length in a longitudinal direction and a width in a transverse direction; and an upper side intended to face a wearer during use of the article and a lower side intended to face away from the wearer during use of the article, wherein the stiffening element extends in a longitudinal direction over at least a part of both a front portion and a crotch portion, and wherein the stiffening element has a width at a transition between the front and crotch portions that is less than the width in the front portion.

The invention is characterized in that the stiffening element comprises a material exhibiting mechanical fastening properties, wherein at least a part of both the upper side and the lower side of the stiffening element exhibits said mechanical fastening properties.

The term mechanical fastening properties as used herein refers to mechanical fastening means such as hooks, friction adhesives, clips, friction elements and combinations thereof, which fastening means as such are well known to the person skilled in art and that allow for a detachable attachment to e.g. surface material of absorption members and/or undergarments of an absorbent article wearer. Thus, a material exhibiting mechanical fastening properties comprises mechanical fastening means of the abovementioned type(s).

Because the inventive stiffening element exhibit said mechanical fastening properties, it can be used as a separate, reusable element when arranged in association with the absorbent article. The upper side of the stiffening element is preferably attached to a lower side of an absorbent member which will be facing the wearer during use. The lower side of the stiffening element can be attached either to the undergarments of the wearer of the article as to keep the article in place during use, or to an upper side of a secondary, lower absorption member as to form a two-part absorbent product comprising an upper and a lower absorption member.

The term absorption member as used herein refers to an item that comprises an absorption body for absorption of body fluids. The absorption member may also comprise e.g. a liquid permeable top-sheet arranged on the upper side of the absorption body and a liquid-impermeable back sheet arranged on the lower side of the absorption body.

The inventive stiffening element has the advantage that, when arranged in an absorbent article, it provides the article with a good body fit and at the same time it allows detachable attachment to the items, i.e. absorbent member(s) and user undergarment, it is attached to. Because the stiffening element provides the article with a suitable shape, it is not necessary that the absorption member contributes to the shaping of the article. Thereby it becomes possible to use a very flexible absorption member, i.e. a very flexible absorption body, which, at least with regard to the absorption member facing the wearer, improves the comfort of the absorbent article. Further, it becomes possible to use simple and non-expensive absorption members. Because of the mechanical fastening properties of the stiffening element, a soiled absorption member can easily be removed and replaced. This is useful also for two-part products since in many situations only the upper absorption member will be soiled after use of the article, in which situations only the upper absorbent member will need to be disposed and replaced. Re-use of the stiffening element is generally possible.

The possibility of attaching the absorbent article, via the inventive stiffening element, to the garments is useful also for articles with advanced body fit, such as the one disclosed in WO 98/22061, because also such articles may move in relation to the wearer during cycling or other vigorous activities.

The possibility of attaching the inventive stiffening element to both an upper, primary absorption member and a lower, secondary absorption member placed below the one facing the wearer is useful for providing a two-part absorbent product that, for instance, may be designed to have a larger wetting area and/or a higher absorption capacity than the corresponding one-part product (i.e. the upper absorption member). Thus, the stiffening element according to the invention enables the use of a convertible article that, if the user so wishes, can be converted to a two-part product at certain occasions.

The inventive stiffening element has a multifunction: shaping and fastening of an absorbent article. Such multifunction is generally useful for decreasing the number of components in the absorbent article which makes the manufacture more effective. In the absorbent article disclosed in EP 1395218 multifunction is achieved by providing an absorbent body with a certain stiffness, i.e. stiffening (shaping) and absorbing properties are combined in the same element. However, stiffening and absorbing properties are not easily combined and such a combined element is rather costly. In contrast, the stiffening (shaping) element of the present invention is a separate item in relation to the absorption element and instead of combining stiffening and absorbing properties it combines stiffening and fastening properties which are easier to combine. For instance, improvement of the fastening properties of a piece of material does not normally lead to any corresponding impairment of the stiffening properties. Further, because the absorbing function does not have to be taken into account it is not necessary that the stiffening element has a shape in the lateral plane of the article that is adapted to this function; i.e. the stiffening element secured to the lower side of the absorption member in the inventive way may comprise e.g. rectangular parts to reduce wastage in the production.

In an advantageous embodiment of the invention the material exhibiting mechanical fastening properties also contributes significantly to the stiffness of the stiffening element. This means that the same material provides both the fastening function and, at least a great deal of, the shaping/stiffening function. This way, the structure of the stiffening element can be simplified in that the need for using particular stiffening components or layers is diminished, or even eliminated.

In a further advantageous embodiment of the invention the material exhibiting mechanical fastening properties is a hook material having hooks protruding from at least one of the sides of the stiffening element. Mechanical fasteners in the form of hooks are well known in the field of absorbent products to be suitable for attachment to textile materials such as undergarments or a non-woven material of an absorption member. Preferably, the hook material comprises a hook carrier layer to which layer the hooks are secured, wherein the hook material significantly contributes to the stiffness of the stiffening member. Thereby, the hook material provides both the fastening and the stiffening/shaping functions. In a variant of this embodiment, the hook material constitutes the stiffening element. With such a design, no further materials are needed for providing the stiffening element with said functions.

In another advantageous embodiment of the invention the material exhibiting mechanical fastening properties is a friction adhesive material. Thus, the surface of the material exhibits a stickiness that is useful for attaching the stiffening element to e.g. undergarments or a back sheet of an upper absorption member. Also friction adhesive materials are well known to the person skilled in the art. In a variant of this embodiment, the friction adhesive material constitutes the stiffening element. With such a design, no further materials are needed for providing stiffening element with said functions.

In another advantageous embodiment of the invention the stiffening element forms a separate item that is detachably attachable to an absorbent member of the absorption article. This allows e.g. easy use and re-use of the stiffening element. Moreover, manufacture can be simplified since it is not necessary to integrate the stiffening member with other parts of the absorbent article.

In another advantageous embodiment of the invention the width at the transition between the front and crotch portions is within the range of 15-45 mm. Preferably, the stiffening element provides, at least when arranged in association with an absorbent article and during use of said article, a three-dimensional bowl-like shape in an area in the front portion. Preferably, the stiffening element has a length in the crotch portion within the range of 70-120 mm. Preferably, side edges of the stiffening element, in the direction from the crotch portion in over the front portion, form an acute angle α with a line in the longitudinal direction of the stiffening element. Preferably, the stiffening element extends further in the longitudinal direction some way in over a rear portion and has a wedge-shaped cutout extending from a rear end edge of the stiffening element in a direction towards the crotch portion.

In a variant of the invention the stiffening element is arranged to allow through-flow of body fluids from its upper side to its lower side.

In another advantageous embodiment of the invention the stiffening element forms part of a larger piece of material from which it can be separated, wherein said piece of material comprises a marking corresponding to a peripheral edge of the stiffening element. This allows for a cost-effective manufacture since the larger piece of material may have any shape suitable for production, e.g. rectangular, and since only a marking needs to be provided. A user can easily cut out the stiffening element by herself. Preferably, a plurality of markings are provided corresponding to different sizes and/or geometries of the stiffening element so that a user can select the most suitable.

The invention also concerns an absorbent article comprising a stiffening element of the type described above.

### BRIEF DESCRIPTION OF DRAWINGS

In the description of the invention given below reference is made to the following figure, in which:
- Figure 1: shows, in a schematic, sectional side view, a first preferred embodiment of the invention,
- Figure 2: shows the underside of an adsorbent article according to another preferred embodiment of the invention, and
- Figure 3: shows the underside of an adsorbent article according to still another preferred embodiment of the invention.

### EMBODIMENTS OF THE INVENTION

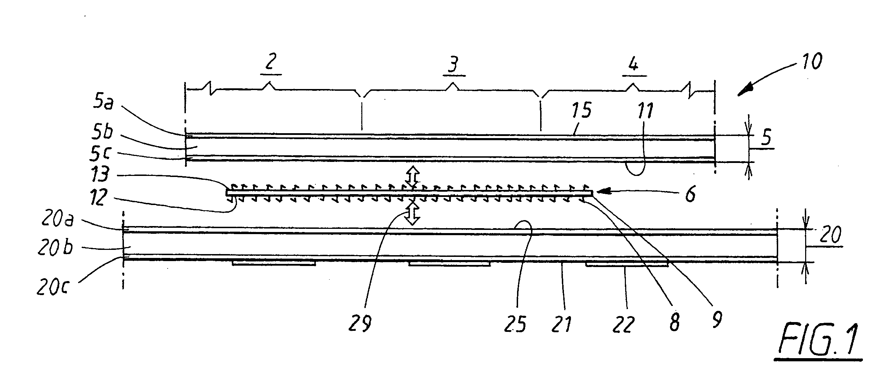

Figure 1 shows, in a schematic, sectional, cut side view, and in a disassembled state, a first preferred embodiment of an absorbent article 1, in this case a sanitary napkin, that includes a stiffening element 6 according to the invention. The absorbent article 1 and the stiffening element 6 have a longitudinal direction, i.e. the left-right direction in figure 1, and a transverse direction, i.e. a direction perpendicular to the plane of figure 1. Further, the article 1 and the stiffening element 6 have a front portion 2, a rear portion 4 and a crotch portion 3 located between the front portion 2 and the rear portion 4. The division of the article 1 and stiffening element 6 into these portions is not strict but describes, in a conventional way, the intended positioning of the article 1 and stiffening element 6 in relation to a wearer.

The absorbent article 1 shown in figure 1 comprises an upper, primary absorbent member 5, the stiffening element 6 and a lower, secondary absorption member 20. The upper absorption member 5 is, via a lower side 11, detachably attachable to the stiffening element 6. Similarly, the lower absorption member 20 is, via an upper side 25, detachably attachable to the stiffening element 6. Thus, the upper and lower absorbent members 5, 20 and the stiffening element 6 are separate components that are detachably attachable such as to form a two-part product with two absorption members. In figure 1 the absorbent article 1 is disassembled. Arrows 29 indicate that the two absorption members 5, 20 may be detachably attached to each other via the stiffening element 6.

The lower absorption member 20 has an upper side 25 intended to face towards a wearer during use of the article 1 and a lower side 21 intended to face away from the wearer during use of the article 1. The lower absorption member 20 exemplified in figure 1 is in principal structured in a conventional way and comprises a liquid-permeable top-sheet 20a, an absorbent body 20b for absorbing body fluids, and a liquid-impermeable back sheet 20c. The top-sheet 20a and the back sheet 20c are interconnected around an outer edge of the absorbent body 20b such as to form a cover around the absorbent body 20b (not shown in figure 1).

The lower side 21 of the secondary absorption member 20 is provided with fastening means 22, for instance in the form of adhesives, for attaching the absorbent article 20 to the undergarments of a user. The fastening means 22 are optional.

Suitable materials and material combinations for forming the top-sheet 20a, the absorbent body 20b and the back sheet 20c are well known to the person skilled in the art. Examples of suitable materials are non-woven fabrics and perforated plastic films for the top-sheet 20a; cellulose fibers, absorbing foam material and super absorbants (SAP) for the absorbent body 20b; and polyethylene film and non-woven fabrics treated with hydrophobing agents for the back sheet 20c.

Which material to choose for the top-sheet 20a depends for instance on which type of mechanical fastening means the stiffening element 6 is provided with. This is further described below.

The stiffening element 6 extends longitudinally and transversely between the upper and lower absorbent members 5, 20 and has an upper side 13 facing the upper absorbent member 5 and a lower side 12 facing the lower absorbent member 20.

In the example shown in figure 1, the stiffening element 6 is entirely made of a hook material comprising a hook carrier layer (hook substrate) 9 and a plurality of hooks 8 secured to the hook carrier layer 9. The hook carrier layer 9 extends longitudinally and transversely. The hooks 8 protrude at least partly in a vertical direction from both sides of the hook carrier layer 9. This means that the hooks 8 protrude from both the lower and upper sides 12, 13 of the stiffening element 6. Hooks constitute one example of mechanical fasteners that are suitable for detachable attachment to textile materials. Thus, the stiffening element 6 in figure 1 is made of a material that exhibits mechanical fastening properties, wherein at least a part of both the upper side 13 and the lower side 12 of the stiffening element 6 exhibit said mechanical fastening properties. Other mechanical fasteners that may be used are clips or friction elements. Combinations of different types of mechanical fasteners may also be used.

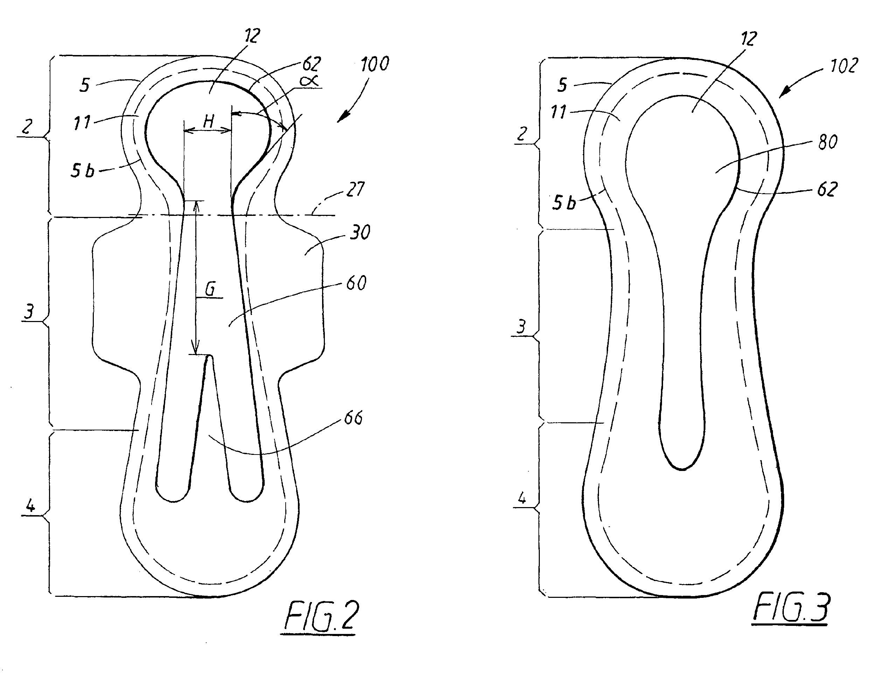

As can be seen in figure 1, the stiffening element 6 extends longitudinally from the front portion, over the crotch portion 3 and into the rear portion 4. This is similar to what is described in relation to figures 2 and 3. The purpose of figure 1 is, however, only to give a schematic view of the structure of the absorbent article. Different shapes of the stiffening element are described below.

The upper absorption member 5 has a similar structure as the lower absorption member 20, i.e. it comprises a liquid-permeable top-sheet 5a, an absorbent body 5b for absorbing body fluids, and a back sheet 5c. However, the back sheet 5c of the upper absorption member 5 is liquid permeable to allow through-flow of body fluids towards the lower absorbent member 20. The top-sheet 5a and the back sheet 5c are interconnected around an outer edge of the absorbent body 5b such as to form a cover around the absorbent body 5b (not shown in figure 1). In this case the back sheet 5c is made of non-woven fabrics which allows for a good attachment to the hooks 8. A back sheet made of e.g. foam or airlaid also allows for a good attachment.

If no secondary absorption member is used, i.e. if the lower side 12 of the stiffening element 6 instead is used to attach the article 10 to the undergarments of a user, it is, of course, suitable to provide the upper absorbent member 5 with a liquid-tight back sheet 5b.

In the example shown in figure 1, also the top-sheet 20a of the lower absorbent member 20 is made of non-woven fabrics to allow for a good attachment with the hooks 8.

The stiffening element 6 can be made liquid permeable by providing it with through-holes distributed in various patterns longitudinally and transversely over the stiffening element 6. This allows through-flow of bodily fluids towards the lower absorption member 20. Alternatively, or in combination, the stiffening element 6 can be made in a liquid-permeable material.

As can be seen in figure 1, the secondary absorption member 20 extends further in the longitudinal direction (i.e. sideways in figure 1) than does the primary, upper absorption member 5. The secondary absorption member 20 also extends further in the transversal direction than does the primary, upper absorption member 5. Accordingly, the secondary absorption member 20 surrounds the primary, upper absorption member 5 as seen from above, i.e. the secondary absorption member 20 exhibits a larger area as seen in a direction towards a user of the absorbent article 20 than does the primary, upper absorption member 5. In short, in the example shown the secondary absorption member 20 is both longer and wider than the upper absorption member 5. This is useful for increasing the leakage protection.

With regard to figure 1 it should be noted that it is not necessary that the back sheet 5c and the stiffening element 6 are liquid permeable to allow body fluids to be transferred to the secondary absorption member 20. If either or both of the back sheet 5c and the stiffening element 6 are liquid impermeable, body fluids may flow transversely (and longitudinally) and pass an outer edge of either or both of these components and then reach the secondary absorption member 20. At least if the back sheet 5c is liquid impermeable it is of particular importance that the secondary absorption member 20 is wider, and preferably also longer, than the primary, upper absorption member 5.

After use of the absorbent article 1, it is possible to dispose and replace only the upper absorption member 5, i.e. the lower absorption member 20 and the stiffening element 6 can be reused. To avoid unnecessary soiling of the lower absorption member 20 it may be useful to provide the upper absorption member 5 with a liquid impermeable back sheet 5b.

It may be noted that it is not necessary that the upper absorption member 5 is smaller, i.e. less wide and less long, than the lower absorption member 20. However, a smaller absorption member is sufficient in many situations and since less material is required for manufacturing a smaller absorption member it is a cost-advantage to use a smaller upper absorption member 5. The larger lower absorption member 20 can thus be regarded as an additional, safety member.

Suitable hook materials for the embodiments shown in figures 1 and 2 are available as KHK0002 or CHK 00752 from 3M Company. These materials have a suitable stiffness for giving the article 1 a good shape during use and provides for a good attachment.

The hooks 8 can, of course, be chosen such as to be adapted to the intended use. That is, the hooks 8 can be adapted to interact (i.e. attach to, detach from, avoid destruction etc.) particularly well with a certain type of material. As an example, both the upper and the lower sides 13, 12 of the stiffening element 6 can comprise hooks 8 particularly adapted to be attached to non-woven fabrics of absorbent members. As a further example, the upper side 13 of the stiffening element 6 can comprise hooks 8 particularly adapted to be attached to non-woven fabrics of an (upper) absorbent member, whereas the lower side 12 can comprise hooks 8 particularly adapted to be attached to undergarments made of cotton. A still further example is that the stiffening element 6 is flat in its uncompressed, non-use state and that it has one side that is particularly adapted to interact with cotton textile material and another side particularly adapted to interact with a synthetic textile material, and wherein both sides are capable of interacting sufficiently well with non-woven fabrics of an absorbent member. In such a case, a user can choose which side should be facing down depending on the type of textile material of the undergarments. Preferably, the stiffening element 6 is provided with an indication on the particular adaptation of its sides, for instance a colour marking, so that a user easily can see how the stiffening element 6 should be positioned.

As an alternative to the hook material described above, the stiffening element 6 can, at least partly, be made of a friction adhesive material, i.e. of a material that exhibits a stickiness that can be used to mechanically fasten the stiffening element 6 to e.g. the upper absorption member 5 and the undergarments. In such a case, the back sheet 5c of the primary absorption member 5 can be made of e.g. a nonwoven material or a perforated plastic film which allows for a good attachment to the friction adhesive material.

Friction adhesive materials, which also are referred to as friction materials, should not be confused with conventional fastening adhesives. A general difference between these materials is that the pressure sensitive adhesive of conventional fastening material does provide reasonable tack, peel and shear after a bond has been initiated by putting pressure onto the system. Different to such a behaviour a friction adhesive material will mainly provide shear. This shear or friction is proportional to the force used to press the friction material onto a second surface. After release of the pressure the system will show basically no remaining tack, peel or friction.

An example of a suitable friction adhesive material is 5401 Traction Tape available from 3M Company.

Hook material and friction adhesive material may be combined in that some parts of the stiffening element 6 comprises hook material and some comprises friction adhesive material. For instance, one side of the stiffening element 6 can comprise hooks and the other friction adhesive material. A mix of hooks and friction adhesive material on the same side is also possible. It is further possible to use a material that has both properties, for instance a hook material that exhibits a stickiness.

Friction adhesive material may be involved when adapting the different sides 12, 13 of the stiffening element 6 to interact particularly well with a certain type of material as described above.

The stiffening element of the invention is sufficiently stiff for, as far as possible, preventing the absorbent article from being compressed or otherwise deformed in an uncontrolled manner during use of the article.

The stiffening element 6 should exhibit a stiffness that is higher than the material of the absorbent article 1 that surrounds the stiffening element. In relation to the embodiment described here, this means that the stiffening element 6 should be stiffer than the upper and lower absorbent members 5, 20. That the stiffness of the stiffening element 6 is higher than its surroundings has the effect that folding indications are provided along and/or around the stiffening element 6. These folding indications, together with e.g. the size and geometry of the stiffening element 6, determine which shape the article 1 will acquire during use.

Preferably, the stiffening element 6 exhibits a stiffness in a dry state in the order of 1-15 N as measured according to ASTM D 4032-82.

The stiffening element 6 can have a variety of shapes and positions depending on the shape desired. Various advantageous shapes of absorbent articles are known to the person skilled in the art. In any case, the stiffening element is arranged to, at least during use of the article, provide the article with a certain, predetermined shape that enhances the fit of the article to the wearer's body. A stiffening element may have a flat form before use but take a three-dimensional shape upon use of the article, i.e. when the article is affected by compressive forces generated by the thighs of the wearer. Alternatively, a stiffening element may have a three-dimensional shape already before use of the article.

Preferably, the stiffening element 6 is arranged such as to, at least during use of the article, provide the article with one or several of the following shapes:
- A width H at a transition 27 (see figure 2) between the crotch portion 3 and the front portion 2 that is less than the width at the front portion 2. This allows anchoring of the article to/between the thigh muscle tendons of the user and prevents the article from moving backwards during use. Preferably, the width H is in the range 15-45 mm.
- A three-dimensional bowl-like shape in an area in the front portion 2. This enhances the body fit.
- A ridge-shaped elevation that partially extends between the wearer's buttocks during use of the article. This prevents rearward leakage.
- A raised portion (hump) intended to make contact with the genitals of the wearer during use of the article. This provides for better absorption of bodily fluids.

Figures 2-3 show absorbent articles comprising stiffening elements 60, 80 according to the invention as seen from below with the main purpose of showing examples of preferred geometries of the stiffening element 6. Thus, the lower side 11 of the (upper) absorption member and the lower side 12 of the stiffening element face upwards in these figures. Any lower absorption members are not shown. The position of the absorbent body 5b is indicated with a dashed line.

Figure 2 shows an absorbent article 100 according to the invention with a first example of a suitable shape of a stiffening element 60. In this example the stiffening element 60 is flat and has a shape similar to what is shown in EP 1395218. A peripheral edge of the stiffening element 60 is indicated by the reference number 62.

Main features of the stiffening element 60, besides the flatness, are inter alia: i) that it extends in the longitudinal direction of the article 100 over the crotch portion 3 and at least some way in over the front portion 2; ii) that it has a width H at the transition 27 between the crotch and front portions 3, 2 that is within the range of 15-45 mm; iii) that it has a length G in the crotch portion 3 within the range of 70-120 mm; iv) that the side edges of the stiffening element 60, in the direction from the crotch area in over the front portion 2, form an acute angle α (i.e. < 90°, preferably 35-55°) with a line in the longitudinal direction of the article 100; and v) that it also extends some way in over the rear portion 4 and has a wedge-shaped cutout 66 extending from a rear end edge of the stiffening element 60 in a direction towards the crotch portion 3, as a result of which the product is during use imparted a fold along the longitudinal direction of the article 100 in said cutout 66, which fold extends into the cleft between the buttocks of the wearer during use of the article 100. These features all contribute to the fit of the article to the wearer during use. One or several of these features may be used to enhance the fit of the article. This means for instance that the stiffening element 60 not necessarily has to extend in over the rear portion 4 and, accordingly, that it ends somewhere in the crotch portion 3 and that there is no cutout 66.

As shown in figure 2, the inventive absorption article 100 may be provided with fastening wings 30 provided with adhesives (not shown in figure 2) for enhanced attachment of the article to the undergarments of the wearer. Such wings are well known to the person skilled in the art.

Figure 3 shows, in a schematic view, an absorbent article 102 according to the invention with a second example of a suitable shape of a stiffening element 80. In this case the stiffening element 80 has the shape of a spoon with a three-dimensional bowl-shaped part located in the front portion 2 and a more narrow part extending longitudinally and centrally over the crotch portion 3, where a hump is formed, and somewhat into the rear portion 4 as to produce a rearward leakage protection as described above.

The invention is not limited by the embodiments described above but can be modified in various ways within the scope of the claims. For instance, the inventive stiffening element 6, 60, 80, irrespective of whether it comprises hook material, friction adhesive material or a combination thereof, does not necessarily have to be made entirely of this or these materials. The stiffening element may e.g. comprise layers of different materials that together build up the total stiffness. What is important is that the stiffness is sufficient for giving the absorbent article a suitable, predetermined shape during use and that the lower and upper sides 12, 13 of the stiffening element 6, 60, 80 exhibit mechanical fastening properties.

The stiffening element 6, 60, 80 and the upper and lower absorption members 5, 20 may be packaged or sold separately and be assembled by a user prior to use. Either or both of the upper and lower absorption members 5, 20 is/are preferably provided with markers for proper positioning of the stiffening element 6. For instance, the lower side 11 of the upper absorption member 5 can be provided with a marker in the form of a line corresponding to the peripheral edge 62 of the stiffening element 6, 60, 80.

The stiffening element 60, 80 can form part of a larger piece of material from which it can be separated, e.g. by cutting. Said piece of material preferably comprises a marking corresponding to the peripheral edge 62 of the stiffening element 6, 60, 80 for simplifying separation of a stiffening element with proper geometry. Such a piece of material can include several markings corresponding to different sizes of stiffening elements 6, 60, 80.

The stiffening element 6, 60, 80 may be articulated for increasing longitudinal flexibility of the article. "Hinges" for this purpose can be arranged in the form of longitudinally distributed slits or hook-free regions if the piece of material making up the stiffening element comprises a hook material.

Foam is an example of a material useful for forming the stiffening element 6, 60,80.

With reference to figure 1, it should be noted that it is not necessary to use a hook material having a single hook carrier layer 9 with hooks 8 protruding in opposite directions. Alternatively, it is possible to use e.g. two parts of a hook material having hooks protruding only in one direction, wherein said parts of hook material are arranged "back to back" (carrier layer to carrier layer) with the hooks protruding away from the other part of hook material.

## Claims

1. Stiffening element (6, 60, 80) for an absorbent article (10, 100, 102), such as a sanitary napkin, a panty liner or an incontinence protector,
wherein the stiffening element (6, 60, 80) is designed such as to, at least during use of the article (10, 100, 102), provide the article with a predetermined shape that enhances the fit of the article (10, 100, 102) to a wearer's body,
said stiffening element (6, 60, 80) having:
- a length in a longitudinal direction and a width in a transverse direction; and
- an upper side (13) intended to face towards a wearer during use of the article (1, 100, 102) and a lower side (12) intended to face away from the wearer during use of the article (1, 100, 102),
wherein the stiffening element (6, 60, 80) extends in the longitudinal direction over at least a part of both a front portion (2) and a crotch portion (3), wherein the stiffening element (6, 60, 80) has a width (H) at a transition (27) between the front and crotch portions (2, 3) that is less than the width in the front portion (2),
**characterized in**
**that** the stiffening element (6, 60, 80) comprises a material exhibiting mechanical fastening properties,
wherein at least a part of both the upper side (13) and the lower side (12) of the stiffening element (6, 60, 80) exhibit said mechanical fastening properties.

2. Stiffening element (6, 60, 80) according to claim 1,
**characterized in**
**that** the material exhibiting mechanical fastening properties also provides a stiffening/shaping function of the stiffening element (6, 60, 80).

3. Stiffening element (6, 60, 80) according to claim 1 or 2,
**characterized in**
**that** the material exhibiting mechanical fastening properties is a hook material having hooks (8) protruding from at least one of the sides (12, 13) of the stiffening element (6, 60, 80).

4. Stiffening element (6, 60, 80) according to claim 3,
**characterized in**
**that** the hook material comprises a hook carrier layer (9) to which layer the hooks (8) are secured, wherein the hook material provides both the fastening function and the stiffening/shaping function of the stiffening member (6, 60, 80).

5. Stiffening element (6, 60, 80) according to claim 4,
**characterized in**
**that** the hook material constitutes the stiffening element (6, 60, 80).

6. Stiffening element (6, 60, 80) according to claim 1 or 2,
**characterized in**
**that** the material exhibiting mechanical fastening properties is a friction adhesive material.

7. Stiffening element (6, 60, 80) according to claim 6,
**characterized in**
**that** the friction adhesive material constitutes the stiffening element (6, 60, 80).

8. Stiffening element (6, 60, 80) according anyone of the above claims,
**characterized in**
**that** it forms a separate item that is detachably attachable to an absorbent member (5, 20) of the absorption article (10, 100, 102).

9. Stiffening element (6, 60, 80) according anyone of the above claims,
**characterized in**
**that** the width (H) at the transition between the front (2) and crotch (3) portions is within the range of 15-45 mm.

10. Stiffening element (6, 60, 80) according anyone of the above claims,
**characterized in**
**that** it has a length (G) in the crotch portion (3) within the range of 70-120 mm.

11. Stiffening element (6, 60, 80) according anyone of the above claims,
**characterized in**
**that** side edges of the stiffening element (6, 60, 80), in the direction from the crotch portion (3) in over the front portion (2), form an acute angle α with a line in the longitudinal direction of the stiffening element (6, 60, 80).

12. Stiffening element (6, 60, 80) according anyone of the above claims,
**characterized in**
**that** it extends further in the longitudinal direction some way in over a rear portion (4) and has a wedge-shaped cutout (66) extending from a rear end edge of the stiffening element (6, 60, 80) in a direction towards the crotch portion (3),

13. Stiffening element (6, 60, 80) according to anyone of the above claims,
**characterized in**
**that** the stiffening element (6, 60, 80) is arranged to allow through-flow of body fluids from its upper side (13) to its lower side (12)..

14. Stiffening element (6, 60, 80) according to anyone of the above claims,
**characterized in**
it forms part of a larger piece of material from which it can be separated, wherein said piece of material comprises a marking corresponding to a peripheral edge (62) of the stiffening element (6, 60, 80).

15. Absorbent article (10, 100, 102), such as a sanitary napkin, a panty liner or an incontinence protector,
**characterized in**
**that** it comprises a stiffening element (6, 60, 80) according to anyone of the above claims.

16. Absorbent article (10, 100, 102) according to claim 15,
**characterized in**
**that** the stiffening element (6, 60, 80) exhibits a stiffness that is higher than a part of the absorbent article (10, 100, 102) that surrounds the stiffening element (6, 60, 80) when the stiffening element (6, 60, 80) is arranged onto the article (10, 100, 102).

17. Absorbent article (10, 100, 102) according to claim 15 or 16,
**characterized in** '
that the article comprises an absorbent member (5) in turn comprising an absorbent body (5b) for absorbing bodily fluids, wherein the absorbent member (5) has an upper side (15) intended to be facing a wearer during use of the article (10, 100, 102) and a lower side (11) intended to be facing away from the wearer during use of the article (10, 100, 102), and wherein the stiffening element (6, 60, 80) is detachably attachable to the lower side (11) of the absorbent member (5).

## Patentansprüche

1. Versteifungselement (6, 60, 80) für einen saugfähigen Artikel (10, 100, 102) wie beispielsweise eine Binde, eine Slipeinlage oder einen Inkontinenzschutz,
wobei das Versteifungselement (6, 60, 80) so ausgebildet ist, wenigstens während des Gebrauchs des Artikels (10, 100, 102) dem Artikel eine vorgegebene Form zu verleihen, welche die Anpassung des Artikels (10, 100, 102) an einen Körper eines Benutzers verbessert,
welches Versteifungselement (6, 60, 80) besitzt:
- eine Länge in einer Längsrichtung und eine Breite in einer Querrichtung; und
- eine Oberseite (13), die dafür vorgesehen ist, dass sie während des Gebrauchs des Artikels (1, 100, 102) einem Benutzer zugewandt ist, und eine Unterseite (12), die dafür vorgesehen ist, dass sie während des Gebrauchs des Artikels (1, 100, 102) dem Benutzer abgewandt ist,
wobei sich das Versteifungselement (6, 60, 80) in der Längsrichtung über wenigstens einen Teil von sowohl einem Vorderteil (2) als einem Schrittteil (3) erstreckt, wobei das Versteifungselement (6, 60, 80) eine Breite (H) an einem Übergang (27) zwischen dem Vorder- und Schrittteil (2, 3) besitzt, die kleiner als die Breite im Vorderteil (2) ist,
**dadurch gekennzeichnet,**
**dass** das Versteifungselement (6, 60, 80) ein Material umfasst, das mechanische Befestigungseigenschaften aufweist,
wobei wenigstens ein Teil von sowohl der Oberseite (13) als auch der Unterseite (12) des Versteifungselements (6, 60, 80) die mechanischen Befestigungseigenschaften aufweist.

2. Versteifungselement (6, 60, 80) nach Anspruch 1,
**dadurch gekennzeichnet,**
**dass** das mechanische Befestigungseigenschaften aufweisende Material ebenfalls eine Versteifungs-/Formgebungsfunktion des Versteifungselements (6, 60, 80) erstellt.

3. Versteifungselement (6, 60, 80) nach Anspruch 1 oder 2,
**dadurch gekennzeichnet,**
**dass** das mechanische Befestigungseigenschaften aufweisende Material ein Hakenmaterial ist, das aus wenigstens einer der Seiten (12, 13) des Versteifungselements (6, 60, 80) herausragende Haken (8) besitzt.

4. Versteifungselement (6, 60, 80) nach Anspruch 3,
**dadurch gekennzeichnet,**
**dass** das Hakenmaterial eine Hakenträgerschicht (9) umfasst, an welcher Schicht die Haken (8) gesichert sind, wobei das Hakenmaterial sowohl die Befestigungsfunktion als auch die Versteifungs-/Formgebungsfunktion des Versteifungselements (6, 60, 80) erstellt.

5. Versteifungselement (6, 60, 80) nach Anspruch 4,
**dadurch gekennzeichnet,**
**dass** das Hakenmaterial das Versteifungselement (6, 60, 80) bildet.

6. Versteifungselement (6, 60, 80) nach Anspruch 1 oder 2,
**dadurch gekennzeichnet,**
**dass** das mechanische Befestigungseigenschaften aufweisende Material ein Friktions-Klebstoffmaterial ist.

7. Versteifungselement (6, 60, 80) nach Anspruch 6,
**dadurch gekennzeichnet,**
**dass** das Friktions-Klebstoffmaterial das Versteifungselement (6, 60, 80) bildet.

8. Versteifungselement (6, 60, 80) nach einem der vorhergehenden Ansprüche,
**dadurch gekennzeichnet,**
**dass** es einen einzelnen Gegenstand ausbildet, der an einem saugfähigen Element (5, 20) des Absorptionsartikels (10, 100, 102) lösbar befestigbar ist.

9. Versteifungselement (6, 60, 80) nach einem der vorhergehenden Ansprüche,
**dadurch gekennzeichnet,**
**dass** die Breite (H) am Übergang zwischen dem Vorder- (2) und Schrittteil (3) innerhalb des Bereichs von 15-45 mm liegt.

10. Versteifungselement (6, 60, 80) nach einem der vorhergehenden Ansprüche,
**dadurch gekennzeichnet,**
**dass** es eine Länge (G) im Schrittteil (3) innerhalb des Bereichs von 70-120 mm besitzt.

11. Versteifungselement (6, 60, 80) nach einem der vorhergehenden Ansprüche,
**dadurch gekennzeichnet,**
**dass** Seitenkanten des Versteifungselements (6, 60, 80) in der Richtung des Schrittteils (3) über den Vorderteil (2) einen spitzen Winkel α mit einer Linie in der Längsrichtung des Versteifungselements (6, 60, 80) bilden.

12. Versteifungselement (6, 60, 80) nach einem der vorhergehenden Ansprüche,
**dadurch gekennzeichnet,**
**dass** es sich weiter in der Längsrichtung auf eine Art über einen hinteren Teil (4) erstreckt und einen keilförmigen Ausschnitt (66) besitzt, der sich von einer hinteren Endkante des Versteifungselements (6, 60, 80) in einer Richtung zum Schrittteil (3) hin erstreckt,

13. Versteifungselement (6, 60, 80) nach einem der vorhergehenden Ansprüche,
**dadurch gekennzeichnet,**
**dass** das Versteifungselement (6, 60, 80) so angeordnet ist, dass es eine Durchströmung von Körperflüssigkeiten von seiner Oberseite (13) an seine Unterseite (12) ermöglicht.

14. Versteifungselement (6, 60, 80) nach einem der vorhergehenden Ansprüche,
**dadurch gekennzeichnet,**
**dass** es einen Teil eines größeren Stücks von Material bildet, von dem es trennbar ist, wobei das Stück von Material eine einer Umfangskante (62) des Versteifungselements (6, 60, 80) entsprechende Markierung umfasst.

15. Saugfähiger Artikel (10, 100, 102) wie beispielsweise eine Binde, eine Slipeinlage oder ein Inkontinenzschutz,
**dadurch gekennzeichnet,**
**dass** er ein Versteifungselement (6, 60, 80) nach einem der vorhergehenden Ansprüche umfasst.

16. Saugfähiger Artikel (10, 100, 102) nach Anspruch 15,
**dadurch gekennzeichnet,**
**dass** das Versteifungselement (6, 60, 80) eine Steifigkeit aufweist, die höher als ein Teil des saugfähigen Artikels (10, 100, 102) ist, der das Versteifungselement (6, 60, 80) umgibt, wenn das Versteifungselement (6, 60, 80) am Artikel (10, 100, 102) angebracht ist.

17. Saugfähiger Artikel (10, 100, 102) nach Anspruch 15 oder 16
**dadurch gekennzeichnet,**
**dass** der Artikel ein saugfähiges Element (5) umfasst, das wiederum einen saugfähigen Körper (5b) zum Absorbieren von Körperflüssigkeiten umfasst, wobei das saugfähige Element (5) eine Oberseite (15), die dafür vorgesehen ist, dass sie während des Gebrauchs des Artikels (10, 100, 102) dem Benutzer zugewandt ist, und eine Unterseite (11), die dafür vorgesehen ist, dass sie während des Gebrauchs des Artikels (10, 100, 102) dem Benutzer abgewandt ist, besitzt, und wobei das Versteifungselement (6, 60, 80) an der Unterseite (11) des saugfähigen Elements (5) lösbar befestigbar ist.

## Revendications

1. Elément raidisseur (6, 60, 80) pour un article absorbant (10, 100, 102), tel qu'une serviette hygiénique, un protège-slip ou une protection contre l'incontinence,
l'élément raidisseur (6, 60, 80) étant conçu de manière, au moins pendant l'utilisation de l'article (10, 100, 102), à pourvoir l'article d'une forme prédéterminée qui améliore l'ajustement de l'article (10, 100, 102) au corps d'un porteur,
ledit élément raidisseur (6, 60, 80) ayant:
- une longueur dans la direction longitudinale et une largeur dans la direction transversale; et
- une face supérieure (13) destinée à être tournée vers un utilisateur pendant l'utilisation de l'article (1, 100, 102), et une face inférieure (12) destinée à être détournée de l'utilisateur pendant l'utilisation de l'article (1, 100, 102), l'élément raidisseur (6, 60, 80) s'étendant dans la direction longitudinale sur au moins une partie à la fois d'une partie avant (2) et d'une partie d'entrejambe (3), l'élément raidisseur (6, 60, 80) ayant une largeur (H) à une transition (27) entre les parties avant et d'entrejambe (2, 3) qui est inférieure à la largeur de la partie avant (2),
**caractérisé en ce que**
l'élément raidisseur (6, 60, 80) comprend un matériau présentant des propriétés mécaniques de fixation,
au moins une partie à la fois de la face supérieure (13) et de la face inférieure (12) de l'élément raidisseur (6, 60, 80) présentant des propriétés mécaniques de fixation.

2. Elément raidisseur (6, 60, 80) selon la revendication 1,
**caractérisé en ce que**
le matériau présentant des propriétés mécaniques de fixation également fournit une fonction de raidissement/formation de l'élément raidisseur (6, 60, 80).

3. Elément raidisseur (6, 60, 80) selon la revendication 1 ou 2,
**caractérisé en ce que**
le matériau présentant des propriétés mécaniques de fixation est un matériau à crochets ayant des crochets (8) faisant saillie à partir d'au moins l'une des faces (12, 13) de l'élément raidisseur (6, 60, 80).

4. Elément raidisseur (6, 60, 80) selon la revendication 3,
**caractérisé en ce que**
le matériau à crochets comprend une couche de support à crochets (9), couche à laquelle les crochets (8) sont fixés, le matériau à crochets fournit à la fois la fonction de fixation et la fonction de raidissement/formation de l'élément raidisseur (6, 60, 80).

5. Elément raidisseur (6, 60, 80) selon la revendication 4,
**caractérisé en ce que**
le matériau à crochets constitue l'élément raidisseur (6, 60, 80).

6. Elément raidisseur (6, 60, 80) selon la revendication 1 ou 2,
**caractérisé en ce que**
le matériau présentant des propriétés mécaniques de fixation est une matière adhésive à friction.

7. Elément raidisseur (6, 60, 80) selon la revendication 6,
**caractérisé en ce que**
la matière adhésive à friction constitue l'élément raidisseur (6, 60, 80).

8. Elément raidisseur (6, 60, 80) selon l'une quelconque des revendications précédentes,
**caractérisé en ce qu'**
il forme un élément distinct qui est fixé de manière amovible à un élément absorbant (5, 20) de l'article absorbant (10, 100, 102).

9. Elément raidisseur (6, 60, 80) selon l'une quelconque des revendications précédentes,
**caractérisé en ce que**
la largeur (H) à la transition entre la partie avant (2) et la partie d'entrejambe (3) est comprise entre 15 et 45 mm.

10. Elément raidisseur (6, 60, 80) selon l'une quelconque des revendications précédentes,
**caractérisé en ce qu'**
il a une longueur (G) dans la partie d'entrejambe (3) comprise entre 70 et 120 mm.

11. Elément raidisseur (6, 60, 80) selon l'une quelconque des revendications précédentes,
**caractérisé en ce que**
des bords latéraux de l'élément raidisseur (6, 60, 80), dans la direction de la partie d'entrejambe (3) au-dessus de la partie avant (2), forment un angle aigu a avec une ligne dans la direction longitudinale de l'élément raidisseur (6, 60, 80).

12. Elément raidisseur (6, 60, 80) selon l'une quelconque des revendications précédentes,
**caractérisé en ce qu'**
il s'étend davantage dans la direction longitudinale à une distance au-dessus d'une partie arrière (4) et présente une découpe cunéiforme (66) s'étendant depuis un bord d'extrémité arrière de l'élément raidisseur (6, 60, 80) dans une direction vers la partie d'entrejambe (3).

13. Elément raidisseur (6, 60, 80) selon l'une quelconque des revendications précédentes,
**caractérisé en ce que**
l'élément raidisseur (6, 60, 80) est agencé de manière à permettre un écoulement de fluides corporels à partir de sa face supérieure (13) à sa face inférieure (12).

14. Elément raidisseur (6, 60, 80) selon l'une quelconque des revendications précédentes,
**caractérisé en ce qu'**
il fait partie d'une pièce de matériau supérieure à partir de laquelle il peut être séparé, ladite pièce de matériau comprenant un marquage correspondant à un bord périphérique (62) de l'élément de raidisseur (6, 60, 80).

15. Article absorbant (10, 100, 102), tel qu'une serviette hygiénique, un protège-slip ou une protection contre l'incontinence,
**caractérisé en ce qu'**
il comprend un élément raidisseur (6, 60, 80) selon l'une quelconque des revendications précédentes.

16. Article absorbant (10, 100, 102) selon la revendication 15,
**caractérisé en ce que**
l'élément raidisseur (6, 60, 80) présente une rigidité qui est supérieure à une partie de l'article absorbant (10, 100, 102) qui entoure l'élément raidisseur (6, 60, 80) lorsque l'élément raidisseur (6, 60, 80) est disposé sur l'article (10, 100, 102).

17. Article absorbant (10, 100, 102) selon la revendication 15 ou 16,
**caractérisé en ce que**
l'article comprend un élément absorbant (5) comprenant à son tour un corps absorbant (5b) destiné à absorber des fluides corporels, l'élément absorbant (5) présentant une face supérieure (15) destinée à être tournée vers un utilisateur pendant l'utilisation de l'article (10, 100, 102) et une face inférieure (11) destinée à être détournée de l'utilisateur pendant l'utilisation de l'article (10, 100, 102), et l'élément raidisseur (6, 60, 80) étant fixé de manière amovible à la face inférieure (11) de l'élément absorbant (5).
